# EUROPEAN PATENT SPECIFICATION

(11) **EP 4 014 973 B1**
(45) Date of publication and mention of the grant of the patent: **23.04.2025**
(21) Application number: 20020630.8
(22) Date of filing: 17.12.2020
(51) Int. Cl.: A61K 31/167, C07C 233/00, A61P 31/04, C07C 43/23, C07C 237/04, C07C 231/12

(54) **INHALED LIQUID FOR THE TREATMENT OF RESPIRATORY INFECTIONS IN HUMANS CAUSED BY PATHOGENS RESISTANT TO ANTIBIOTICS**
INHALATIONSFLÜSSIGKEIT ZUR BEHANDLUNG VON DURCH ANTIBIOTIKARESISTENTE ERREGER VERURSACHTE ATEMWEGSINFEKTIONEN BEIM MENSCHEN
LIQUIDE INHALÉ POUR LE TRAITEMENT DES INFECTIONS RESPIRATOIRES CHEZ L'HOMME CAUSÉES PAR DES AGENTS PATHOGÈNES RÉSISTANTS AUX ANTIBIOTIQUES

(43) Date of publication of application: 22.06.2022
(73) Proprietor: Conick Biotech LLC, San Diego, CA 92127 (US)
(72) Inventor: Ninkov, Dusan, 1066 Budapest (HU)
(74) Representative: Filipova, Liljana

(56) References cited:
- WO-A1-03/049726
- WO-A1-2019/028937
- US-A1- 2003 176 364
- WIMBERLEY NEIL ET AL: "Antibacterial Properties of Lidocaine", CHEST, vol. 76, no. 1, 1 July 1979 (1979-07-01), US, pages 37 - 40, XP055801501, ISSN: 0012-3692, Retrieved from the Internet <URL:http://dx.doi.org/10.1378/chest.76.1.37> DOI: 10.1378/chest.76.1.37
- SAMET MOHAMMAD ET AL: "Effect of Lidocaine 2% on Bacterial Culture of Bronchial Fluid", JOURNAL OF THE COLLEGE OF PHYSICIANS AND SURGEONS PAKISTAN, 1 January 2017 (2017-01-01), pages 771 - 774, XP055801465, Retrieved from the Internet <URL:https://www.jcpsp.pk/archive/2017/Dec2017/09.pdf> [retrieved on 20210504]

## Description

### FIELD OF THE INVENTION

The compound is a novel medicine for the treatment of respiratory infections in humans.

### BACKGROUND OF THE INVENTION

This invention is addressing the problems of respiratory infections in humans with the different etiology of pathogens. The compound is a broad-spectrum antimicrobial compound in the form of a liquid meant for inhalation for the treatment of respiratory infections. The antimicrobial effect of Eugenol is well known. This invention is completely novel because Eugenol and Lidocaine, by the special process of synthesis have been bounced into one stable compound. The effect and uniqueness of this compound is the subject of this invention. The active principle of the compound is a stable quaternary ammonium complex of Eugenol and Lidocaine in the form of an inhaled liquid. The compound is more effective than standard 3rd generation cephalosporin antibiotics in the treatment of mentioned infections. The compound is created to fight antibiotic resistance of pathogenic bacteria, such as *Pasteurella spp., Cryptococcus spp., Campylobacter spp., Microsporidia spp., Histoplasma capsulatum, Micrococcus spp., Candida spp.,* and other bacteria causing respiratory infections in humans. Based on the specific mechanism of action, the active compound of this invention is different from any other antibiotic currently available on the market. In this invention, an extra potent effect of the synergy of Eugenol and Lidocaine is achieved. The antimicrobial efficacy of this novel compound will be significantly more effective than Eugenol and Lidocaine by themselves or by their simple mixture.

The patent application US 2003/176364 A1 (NINKOV DUSAN [US]) describes the potential of a synthesis of eugenol and lidocaine, our invention has in detail described the much more complex synthesis process than a simple synthesis of procaine and carvacrol. The novel synthesis in our invention represents an entirely novel technological process and has produced a more effective and complex novel compound between eugenol and lidocaine. Also, this process in our invention has proven that eugenol and lidocaine cannot be synthesized into a single new compound by using the process described in the patent application US 2003/176364 A1. Additionally, in some patients, procaine has been shown to cause more allergies as a side effect. The application of this patent was intramuscular, intravenous, etc., but not inhalation.

In the patent application WO 2019/028937 A1 (UNIV FOSHAN [CN]), was stated that eugenol could be used for controlling Pseudomonas aeruginosa, Staphylococcus aureus, Staphylococcus albus, Staphylococcus citreus, group A streptococcus, group B streptococcus, and Candida albicans. Unlike the active compound of this patent which uses clove oil and its active compound eugenol, in our patent, we have a uniquely developed synthesis of eugenol and lidocaine. This unique method of synthesis has produced a novel compound for the inhalation and treatment of various lung infections. In our novel process of synthesis, clove oil has been substituted for a 99% purity of eugenol.

Unlike in patent application WO 03/049726 A1 (XIMED GROUP PLC [US]), the active compound of this invention has been produced as a terpene mixture of liposome terpenes, or the active compound represents a single terpene. The standard concentration of combinations in patent application WO 03/049726 A1 cannot be controlled nor replicated consistently. Although this invention did not describe in detail its mechanism of action, the applications of these four possibilities are applied by inhalation. Along those same lines, our patent application goes in-depth with regards to using a nebulizer, unlike the abovementioned process.

The article (Wimberly Neil et al. July 1979 pages 37-40) describes the antimicrobial properties of lidocaine, and from it, our patent application elaborates on the information provided in the article whereby a unique synthesis process is used to get the desired effects of combing lidocaine and eugenol.

The article (Samet Mohammad et al. (2017-01-01, pages 771-774) describes the positive effect of lidocaine 2% on the bacterial culture of the bronchial fluid in treated patients. Although the positive effect of 2% lidocaine has been proven, our invention is more complex. The novel invention elaborates on the complexity of our patent application. In it, lidocaine with a purity of 99.5% was used as one of the synthesis compounds, representing approximately 44.5% of the solution. The positive effect of lidocaine 2% on patients with lung infections has strongly suggested a correlating factor that our invention will also have a positive effect.

Before the widespread use of Penicillin, infectious diseases were the leading cause of death among all age groups across the globe. Antibiotics have extended the lives and improved the quality of life of people since the early 20th century. Many have thought that humanity was entering the post-pathogen era, where infectious disease was a historical footnote. Today, infective diseases are still the first leading cause of death and the leading cause of disability.

### DESCRIPTION OF THE INVENTION

The present invention is defined by the independent claims. The dependent claims depict other embodiments of the invention. Any embodiment not falling under the scope of the appended claims does not form part of the invention. In the following text, the novelty and uniqueness of the compound will be elaborated.

### Method of production

The novel compound is synthesized by reacting Eugenol (Sigma Aldrich) and an equimolar mass of free base Lidocaine (Sigma Aldrich) in a Dichloromethane solvent. After the reaction of those two chemicals at room temperature, for 30 minutes, a rotary vacuum evaporator is used to evaporate Dichloromethane from the mixture, leaving us with the pure novel compound. The novel compound is a quaternary ammonium complex salt of the naturally existing phenolic compound - Eugenol, with a synthetic local anesthetic compound - Lidocaine, as a free base form. The ratio of Lidocaine to Eugenol in this reaction is in the range between 1:1 and 2:1

### Schematic diagram of synthesis procedure

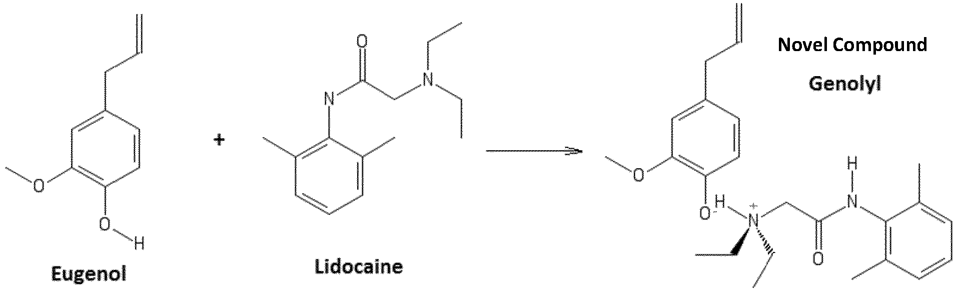

### Structural formula of the compound

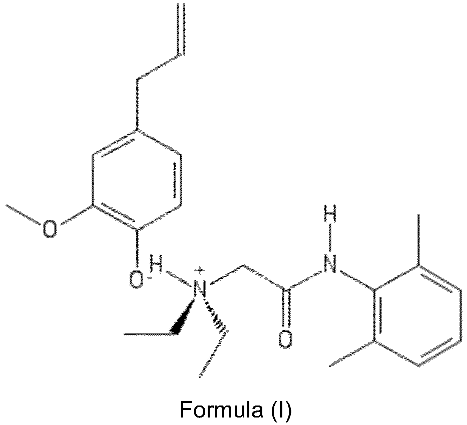

Chemical formula: C₂₄H₃₄N₃O₃

Molecular mass: 398.54 g mol⁻¹

### Compound chemical name:

N-[N'-(carboxymethylenyl-2,6-dimethyl-aniline)-N,N-diethylammonium]- [2'-methoxy-4'-(2'-propenyl)]-phenolate.

### Characterization

Gas-mass and HPLC chromatography can be used to characterize the new complex. In polar solvents such water-methanol systems, two separate peaks of the complex constituents should be obtained, Lidocaine as a cation and Eugenol as an anion. Polar solvents tend to separate anions from cations in solution and that is the reason why two separate peaks in such a system can be observed. Each peak is characterized by a mass spectrometer and the information obtained proves their origin from Eugenol and Lidocaine. The new complex can also be characterized on 1H-NMR and IR spectra.

### Brief description of the Figures

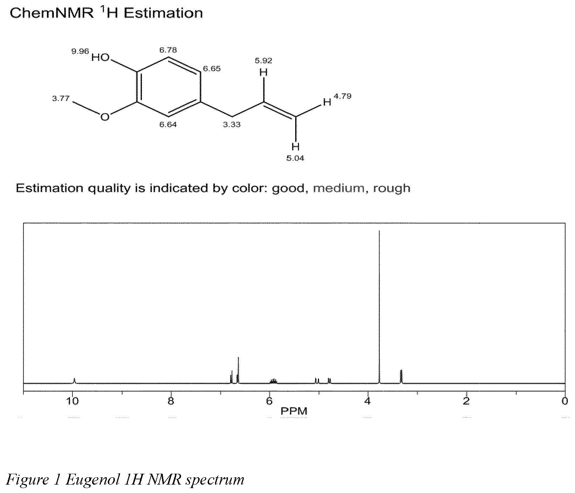

*Figure 1**: Eugenol 1H NMR spectrum analysis, obtained from a reputable chemical database (ChemNMR)*

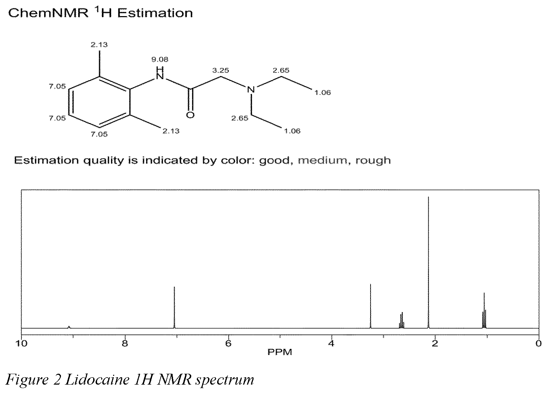

*Figure 2**: Lidocaine 1H NMR spectrum analysis, obtained from a reputable chemical database (ChemNMRI)*

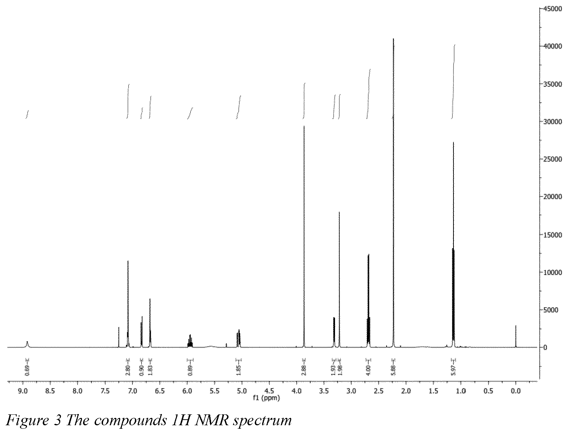

*Figure 3**: The compounds 1H NMR spectrum analysis, obtained after the synthesis of the novel compound.*

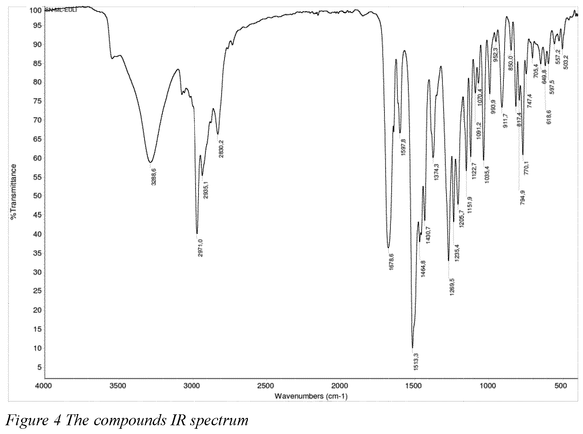

*Figure 4**: The compounds IR spectrum analysis, obtained after the synthesis of the novel compound.*

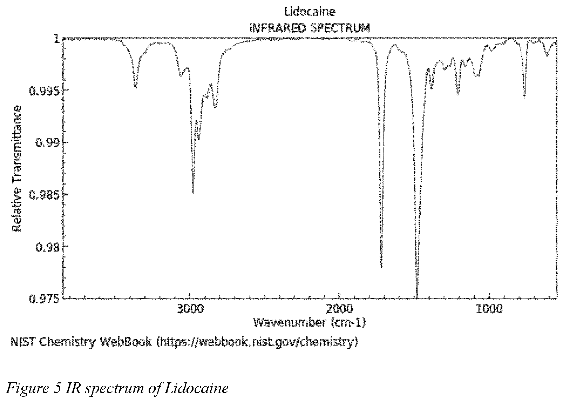

*Figure 5**: Lidocaine IR spectrum analysis, obtained from a reputable chemical database (NIST Chemistry WebBook).*

### Quality control

For quality control, gas-mass chromatography and HPLC can be used. Peaks of Eugenol and Lidocaine are acceptable since the novel compound dissociate producing them. Every other peak in the chromatogram can be considered impure. By measuring the peak area, the percentage of the impurities can be calculated. Using mass spectra or IR spectra, the origin of impurities can be obtained.

### Method of novelty

In 1H-NMR spectra, the difference between free Eugenol characteristic positions of H-atoms and the same position of corresponding H-atoms of free Eugenol from a database was compared. The different positions of those H-atoms would conclude that the obtained compound is a new compound, a combination of Eugenol and Lidocaine. Additionally, the IR spectra of the novel compound was also measured, proving that the novel compound is in fact novel by not having the spectra data in any reputable database.

Eugenol is colorless to pale yellow oily liquid at room temperature. It can be found in many essential oils from natural products such as clove oil, nutmeg, cinnamon, basil bay leaf oil and possess antiseptic and anesthetic properties. It is also a well-known antibacterial, antifungal, antimicrobial and anticoagulant agent. Uses of Eugenol have a wide spectrum and its potential for medical uses is tremendous.

### Manufacturing of the compound

This text is a brief description of how the compound will be manufactured.

### Formula:

| | |
|---|---|
| Water: | 90 ml |
| Emulsifier: | 8 ml |
| Novel active compound: | 2 ml |
| TOTAL: | 100 ml |

The compound was obtained by mixing the above stated ingredients in a vacuum mixer (Silverson L5M-A Sealed Unit Lab Mixer) at 6,000 rpm for 20 minutes.

### Treatment of respiratory infections in humans

- Case no. 1: Y.D., Male, Age 55, USA, Albuquerque Symptoms: Acute Bronchitis
- Case no. 2: I.M., Male, Age 24, USA, Madison Symptoms: Acute Bronchitis
- Case no. 3: F.P., Male, Age 59, USA, Greensboro Symptoms: Pneumonia
- Case no. 4: A.Y., Male, Age 50, USA, Bakersfield Symptoms: Bronchopneumonia
- Case no. 5: J.D., Male, Age 38, USA, Fort Worth Symptoms: Acute Bronchitis
- Case no. 6: D.P., Male, Age 21, USA, Tucson Symptoms: Acute Bronchitis
- Case no. 7: L.R., Male, Age 26, France, Paris Symptoms: Bronchopneumonia
- Case no. 8: G.V., Male, Age 43, USA, Jersey City Symptoms: Pneumonia
- Case no. 9: W.Z. Female, Age 21, USA, Long Beach Symptoms: Pneumonia
- Case no. 10: G.J. Female, Age 34, USA, San Jose Symptoms: Acute Bronchitis
- Case no. 11: W.B. Female, Age 27, Norway, Oslo Symptoms: Bronchopneumonia
- Case no. 12: C.R. Female, Age 56, USA, Los Angeles Symptoms: Pneumonia
- Case no. 13: O.B. Female, Age 34, USA, Rochester Symptoms: Acute Bronchitis
- Case no. 14: Z.F. Female, Age 50, USA, Columbus Symptoms: Bronchopneumonia
- Case no. 15: K.U. Female, Age 32, Canada, Toronto Symptoms: Acute Bronchitis

### Additional information and results of testing

The reason for this testing was to check if the novel compound, after a longer period of use, can cause any eventual side effects.

The test results were generated and monitored in order to obtain the relevant information and facts. In this study, the general monitored parameters were pulse, breathing, ECG (Electrocardiography), any discomfort, adverse events and/or allergy monitoring. These parameters were measured before the treatment with the novel compound and 15 minutes after its intake. The novel compound was ingested on the therapeutic days in each patient at 06:00 AM, 02:00 PM and 10:00 PM (Table 1 and Table 2). After the novel compound had been inhaled, the physician made observations for 30 min. Feeling of the patients during and after the inhalation of the novel compound will also be recorded for each participant

As part of the biochemical analysis of patients' blood, the blood sample of each patient were checked for biochemical parameters. Blood samples were taken from each patient before the beginning of the test, on the 4^{th} day from test beginning, and on the 8^{th} day form test beginning. The last blood samples have to be checked 12 hours from the last inhalation of the novel compound. The biochemical parameters that were measured in blood samples include:
1. Liver status
2. C Reactive protein
3. Kidney Status

The dose was: 10ml of the novel compound 2% / 30 kg of the body weight every 8 hours for seven (7) days (Table 1).

The clinical study started on October 15^{th}, 2017 and was completed on November 21^{th} 2017.

Each patient received a dose at 6:00 AM, 2:00 PM, and 10:00 PM each day for the duration of seven (7) days.

List of participating male patients and the corresponding doses of the novel compound:

| No. | Patient | Gender | Age (years) | Weight (kg) | Dose (ml) |
|---|---|---|---|---|---|
| 1 | J.P. | M | 27 | 71 | 23,67 |
| 2 | I.N. | M | 31 | 72 | 24,00 |
| 3 | M.S. | M | 23 | 63 | 21,00 |
| 4 | P.S. | M | 29 | 67 | 22,33 |
| 5 | I.J. | M | 37 | 74 | 24,67 |
| 6 | A.V. | M | 20 | 66 | 22,00 |
| 7 | P.S. | M | 24 | 70 | 23,33 |
| 8 | R.M. | M | 32 | 73 | 24,33 |
| 9 | D.N. | M | 39 | 68 | 22,67 |
| 10 | N.S. | M | 37 | 71 | 23,67 |
| 11 | D.S. | M | 28 | 69 | 23,00 |
| 12 | L.V. | M | 40 | 73 | 24,33 |
| 13 | N.A. | M | 22 | 63 | 21,00 |
| 14 | S.M. | M | 25 | 67 | 22,33 |
| 15 | T.S. | M | 21 | 65 | 21,67 |
| 16 | O.L. | M | 33 | 66 | 22,00 |
| 17 | Z.M. | M | 29 | 73 | 24,33 |
| 18 | I.S. | M | 25 | 63 | 21,00 |
| 19 | J.T. | M | 40 | 74 | 24,67 |
| 20 | P.L. | M | 37 | 71 | 23,67 |
| 21 | V.J. | M | 24 | 67 | 22,33 |
| 22 | G.N. | M | 21 | 66 | 22,00 |
| 23 | R.S. | M | 36 | 69 | 23,00 |
| 24 | P.J. | M | 20 | 63 | 21,00 |
| 25 | D.V. | M | 33 | 71 | 23,67 |
| 26 | S.I. | M | 28 | 74 | 24,67 |
| 27 | K.D. | M | 22 | 68 | 22,67 |
| 28 | V.I. | M | 39 | 72 | 24,00 |
| 29 | I.A. | M | 31 | 65 | 21,67 |
| 30 | P.T. | M | 35 | 69 | 23,00 |
| 31 | R.V. | M | 27 | 71 | 23,67 |
| 32 | T.N. | M | 20 | 64 | 21,33 |
| 33 | M.A. | M | 26 | 72 | 24,00 |
| 34 | I.R. | M | 21 | 63 | 21,00 |
| 35 | J.V. | M | 29 | 67 | 22,33 |
| 36 | G.S. | M | 33 | 74 | 24,67 |
| 37 | O.D. | M | 28 | 72 | 24,00 |
| 38 | Z.V. | M | 24 | 68 | 22,67 |
| 39 | V.T. | M | 31 | 73 | 24,33 |
| 40 | S.S. | M | 38 | 74 | 24,67 |

List of participating female patients and the corresponding doses of the novel compound:

| No. | Patient | Gender | Age (years) | Weight (kg) | Dose (ml) |
|---|---|---|---|---|---|
| 1 | M.I. | F | 24 | 58 | 19,33 |
| 2 | J.S. | F | 31 | 63 | 21,00 |
| 3 | M.L. | F | 27 | 61 | 20,33 |
| 4 | A.R. | F | 25 | 65 | 21,67 |
| 5 | I.S. | F | 33 | 57 | 19,00 |
| 6 | L.M. | F | 34 | 64 | 21,33 |
| 7 | P.S. | F | 20 | 62 | 20,67 |
| 8 | T.T. | F | 22 | 59 | 19,67 |
| 9 | R.P. | F | 28 | 64 | 21,33 |
| 10 | I.N. | F | 25 | 61 | 20,33 |
| 11 | J.M. | F | 29 | 67 | 22,33 |
| 12 | Z.N. | F | 23 | 56 | 18,67 |
| 13 | S.V. | F | 34 | 62 | 20,67 |
| 14 | LJ.V. | F | 31 | 64 | 21,33 |
| 15 | N.J. | F | 30 | 61 | 20,33 |
| 16 | S.O. | F | 27 | 62 | 20,67 |
| 17 | K.N. | F | 21 | 57 | 19,00 |
| 18 | S.B. | F | 29 | 59 | 19,67 |
| 19 | M.C. | F | 24 | 58 | 19,33 |
| 20 | S.D. | F | 33 | 64 | 21,33 |
| 21 | L.D. | F | 38 | 66 | 22,00 |
| 22 | T.Z. | F | 25 | 62 | 20,67 |
| 23 | P.T. | F | 24 | 67 | 22,33 |
| 24 | L.S. | F | 31 | 61 | 20,33 |
| 25 | E.C. | F | 26 | 58 | 19,33 |
| 26 | A.V. | F | 21 | 61 | 20,33 |
| 27 | D.A. | F | 24 | 68 | 22,67 |
| 28 | R.D. | F | 28 | 71 | 23,67 |
| 29 | I.B. | F | 20 | 62 | 20,67 |
| 30 | O.R. | F | 33 | 64 | 21,33 |
| 31 | K.A. | F | 29 | 68 | 22,67 |
| 32 | I.D. | F | 25 | 61 | 20,33 |
| 33 | P.R. | F | 21 | 57 | 19,00 |
| 34 | E.N. | F | 34 | 66 | 22,00 |
| 35 | U.R. | F | 31 | 68 | 22,67 |
| 36 | A.P. | F | 28 | 65 | 21,67 |
| 37 | Z.A. | F | 23 | 59 | 19,67 |
| 38 | I.G. | F | 21 | 61 | 20,33 |
| 39 | R.B. | F | 29 | 63 | 21,00 |
| 40 | O.K. | F | 31 | 70 | 23,33 |

Based on the results obtained during this clinical pilot study of the compound on 80 humans, the following conclusions can be made:
1. The participants of the study showed tolerance to the novel compound upon the prescribed dosage.
2. There were no symptoms of allergy due to the prescribed dosage of the novel compound.
3. During the inhalation of the novel compound, pulse frequency and blood pressure were not significantly altered.
4. Electrocardiography measurements during the intake of the novel compound were regular without any pathological and irregular oscillations.
5. Respiratory function was also monitored during the clinical study. The respiratory function of patients was not disturbed, and none of the patients reported any irregularity in respiratory function during the study.
6. No dyspnea was recorded during the test.
7. No bronchospasms were recorded.
8. Sporadic coughing was recorded in some participants in the very beginning of the test.
9. No irregular parameters were reported in the liver function biochemical analysis from blood samples during the study. Therefore, the novel compound did not have any negative clinical or acute effects on the hepatic function of participants volunteering in this study.
10. No irregular parameters were reported during the testing of kidney functions for the period of testing the novel compound. The test results of both urine and blood parameters in related to the kidney functions, have suggested that the novel compound did not have any negative effects on kidney of patients.
11. The results of C Reactive Protein (CRP) measurements during the study with the novel compound suggest that no inflammation was developed.
12. After 10 working day from the end of the testing, no irregularities were observed on the tested participants.

### The compound In Vitro test

Here are the findings of the comparative examination of selected pathogens against the compound and the antibiotics Cephalosporin and Ampicillin:

| Bacteria | Method | Novel Compound | R | Cephalosporin | R | Ampicilin | R |
|---|---|---|---|---|---|---|---|
| Pasteurella | Müller-Hinton-Agar | 36 mm | ES | 12 mm | S | 5 mm | MS |
| Cryptococcus | Müller-Hinton-Agar | 31 mm | ES | 6 mm | MS | 0 mm | R |
| Campylobacter | Müller-Hinton-Agar | 32 mm | ES | 11 mm | S | 7 mm | MS |
| Microsporidia | Müller-Hinton-Agar | 30 mm | ES | 22 mm | ES | 3 mm | R |
| Histoplasma capsulatum | Müller-Hinton-Agar | 34 mm | ES | 4 mm | R | 2 mm | R |
| Micrococcus | Müller-Hinton-Agar | 38 mm | ES | 8 mm | MS | 0 mm | R |
| Candida | Müller-Hinton-Agar | 33 mm | ES | 7 mm | MS | 4 mm | R |

Amount used: The novel compound 0.05 mL; Cephalosporin 0.1 mL; Ampicillin 0.2 mL.

Evaluation of the results by zone diameter:
0mm - 4mm: resistant: R
5 - 9 mm: moderately sensitive: MS
10 - 15 mm: sensitive: S
16 - 20 mm: very sensitive: VS
21 mm and more: extremely sensitive: ES

### Testing of the toxicity of the novel compound inhalable liquid

This testing's objective was to determine the acute toxicity - LD50 of the novel compound inhalable liquid in mice and rats. Both mice and rats were specially selected for the testing of the toxicity of the compounds, medicine, and chemicals. Only the testing of acute toxicity was done since the active therapy with the novel compound takes from one (1) to three (3) days. Sub-acute toxicity was not necessary and applicable since extended treatment with the novel compound will not be in function or planned. Testing was done in the inhalable application in both white lab rats and white lab mice. Groups of the 100 lab mice and rats of both genders were placed into glass compartments. They were regularly consuming food manufactured for these lab animals. Water consumption was always available.

Inhalation administration requires more drug than other routs of administration.

In this toxicology test - acute toxicity of the novel compound was tested:
Oral LD50 p.o. - mice
Oral LD 50 p.o. - rats
Inhalation LD50 - mice
Inhalation LD50 - rats

### Peroral application (p.o.) of the novel compound - LD50 - testing and doses

The peroral application (p.o.) acute toxicity LD50 testing was upon the standard procedure: application p.o. with the special lab pipette easy to administer liquid product: orally to the lab animals.

Results of the peroral application (p.o.) testing of LD50 of the novel compound at mice Peroral application (p.o.) of the novel compound testing LD50 has been by mice: 1 µL / kg, 5 µL / kg, 10 µL / kg, 20 µL / kg, 50 µL / kg, 100 µL / kg, 150 µL / kg, 200 µL / kg, 300 µL / kg, 400 µL / kg, 500 µL / kg, 600 µL / kg, 700 µL / kg, 800 µL / kg, 900 µL / kg, 1,000 µL / kg, 1250 µL / kg, 1,500 µL / kg, 1,750 µL / kg and 2,000 µL / kg. At the level of oral dose LD50 2,000 µL / kg - twenty-three (23) mice out of hundred (100), have changed their behavior and demonstrated light discomfort: running as well as anxiety. Breading and cardiac functions were regular, function of thorax was regular and without visual changes. Discomfort symptoms noticed after the applications, were disappeared after 1 - 3 min., diverse from mouse to mouse. No mouse has ended lethally during this test. Conclusion based on these test results was: the novel compound has oral LD50 over 2,000 µL / kg for mice (p.o.). the novel compound is low toxic in the applied doses for mice orally (p.o.).

### Results of the peroral application (p.o.) testing of LD50 of the novel compound at rats

Peroral application (p.o.) of the novel compound testing LD50 has been by rats: 1 µL / kg, 5 µL / kg, 10 µL / kg, 20 µL / kg, 50 µL / kg, 100 µL / kg, 150 µL / kg, 200 µL / kg, 300 µL / kg, 400 µL / kg, 500 µL / kg, 600 µL / kg, 650 µL / kg, 750 µL / kg, 850 µL / kg, 1,000 µL / kg, 1250 µL / kg, 1,500 µL / kg, 2,000 µL and kg, 2,250 µL. At the level of oral dose LD50 2,250 µL / kg - thirty-seven (37) rats out of hundred (100), have changed their behavior and demonstrated light discomfort: running over the glass cubical, as well as anxiety. Breading and cardiac functions were regular, function of thorax was regular and without visual changes. Discomfort symptoms noticed after the applications, were disappeared after 1 - 3 min., diverse from rat to rat. No rats have ended lethally during this test. Conclusion based on these test results was: the novel compound has oral LD50 over 2,250 µL / kg for rat (p.o.). the novel compound is low toxic in the applied doses for rats orally (p.o.).

### Inhalatory application of the novel compound - LD50 - testing and doses

Inhalatory testing of the novel compound LD50 by nose-only exposure, designed for the easy application of the novel compound to the lab animals through inhalation. The inhalation process was conducted using a nebulizer with tubes going directly into the nostrils of the animals. The exposure time for laboratory rats was 6 hours for each testing and 4 hours for laboratory mice.

Results of inhalatory application of the novel compound in mice

Inhalatory application the novel compound testing LD50 has been in mice: 1 µL / kg, 5 µL / kg, 10 µL / kg, 20 µL / kg, 50 µL / kg, 100 µL / kg, 150 µL / kg, 200 µL / kg, 300 µL / kg, 400 µL / kg, 500 µL / kg, 600 µL / kg, 700 µL / kg, 800 µL / kg, 900 µL / kg, 1,000 µL / kg, 1250 µL / kg, 1,500 µL / kg, 1,750 µL / kg, 2,000 µL / kg and 2,250 µL / kg. At the level of i.p. dose LD50 2,250 µL / kg - twenty-one (21) mice out of hundred (100), have changed their behavior and demonstrated discomfort: running as well as anxiety. Breading and cardiac functions were regular, function of thorax were regular and without visual changes. Discomfort symptoms noticed after the applications, were disappeared after 3 - 5 min., diverse from mouse to mouse. No mouse has ended lethally during this test. Conclusion based on these test results was: the novel compound inhailable liquid has LD50 over 2,250 µL / kg for mice. the novel compound is low toxic in the applied doses on mice. The animals were reexamined after 14 days, and no new irregularities were found.

### Results of inhalatory application of the novel compound in rats

Inhalatory application of the novel compound testing LD50 has been by rats: 1 µL / kg, 5 µL / kg, 10 µL / kg, 20 µL / kg, 50 µL / kg, 100 µL / kg, 150 µL / kg, 200 µL / kg, 300 µL / kg, 400 µL / kg, 500 µL / kg, 600 µL / kg, 700 µL / kg, 800 µL / kg, 900 µL / kg, 1,000 µL / kg, 1250 µL / kg, 1,500 µL / kg, 1,750 µL / kg, 2,000 µL / kg , 2,250 µL / kg and 2,500 µL / kg. At the level of i.p. dose LD50 2,500 µL / kg - twenty-nine (29) rats out of hundred (100), were changed their behavior and demonstrated discomfort: running over the glass cubical as well as the anxiety. Breading and cardiac functions were regular, function of thorax was regular and without visual changes. Discomfort symptoms noticed after the applications, were disappeared after 5 - 6 min., diverse from rat to rat. No rat has ended lethally during this test. Conclusion based on these test results was: the novel compound inhalable liquid has LD50 over 2,500 µL / kg for rats. the novel compound is low toxic in the applied doses on rats. The animals were reexamined after 14 days, and no new irregularities were found

### Mutagenic and cancer-genic tests of the novel compound inhalable liquid

The subject of the testing was to determine the mutagenic and carcinogenic effect of the novel compound inhalable liquid has been conducted. The test has been done using the Ames test. This test, which is a widely and pretty often used method that uses pathogens, in most cases bacteria, to test whether a given chemical, compound, and/or medicine can damage the tested organism's DNA. This method is fast to conduct and easy to follow to check the tested compound's mutagenic or carcinogenic effect. The lab dish with a specially selected Candida strain, which requires histidine, was added to a rat liver extract. The test has been done using the control - experimental method. In the dish volume of the 20 ml of liquid considered to be a control one did not have any the novel compound liquid. The dish we consider to be the experimental one, 1ml of the novel compound was added to the 20 ml of liquid in the dish. The mutagenic test results were negative in both lab dishes - control and experimental plates. The results were identical with no signs of the mutations. Based on these preliminary results, a conclusion could be made that the novel compound possesses no mutagenic and/or cancerogenic effects.

## Claims

1. A compound of formula (I) having a structure as shown below:

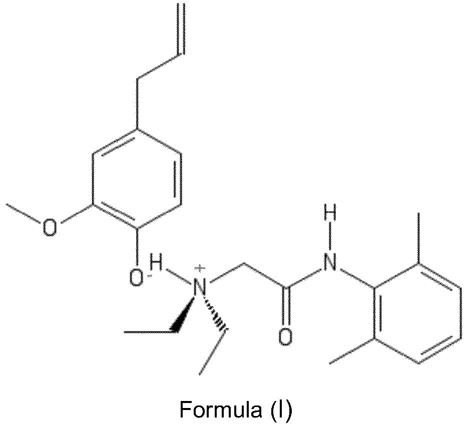

2. The compound , according to the claim 1, as an active compound/potent antimicrobial for use in the treatment of respiratory infections in humans caused by pathogens resistant to antibiotics, such as Pasteurella spp., Cryptococcus spp., Campylobacter spp., Microsporidia spp., Histoplasma capsulatum, Micrococcus spp., Candida spp and other bacteria , in the form of an inhaled liquid.

## Patentansprüche

1. Eine Verbindung der Formel (I) mit der unten dargestellten Struktur:

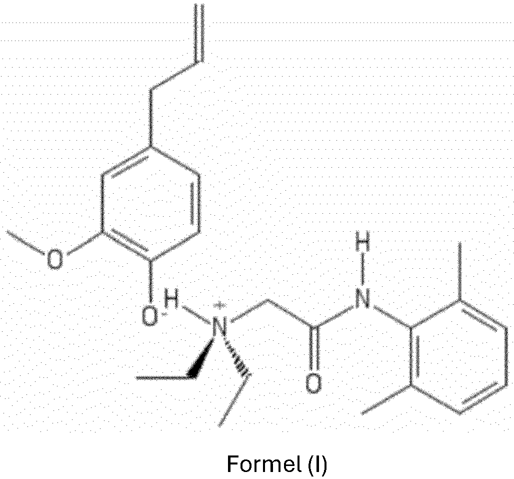

2. Die verbindung gemäß anspruch 1 als wirkstoff/starkes antimikrobielles mittel zur behandlung von atemwegsinfektionen beim menschen, die durch antibiotikaresistente erreger wie Pasteurella spp., Cryptococcus spp., Campylobacter spp., Microsporidia spp., Histoplasma capsulatum, Micrococcus spp., Candida spp. und andere bakterien verursacht werden, in form einer inhalierbaren flüssigkeit.

## Revendications

1. Une combinaison de la formule (I) avec la structure représentée ci-dessous:

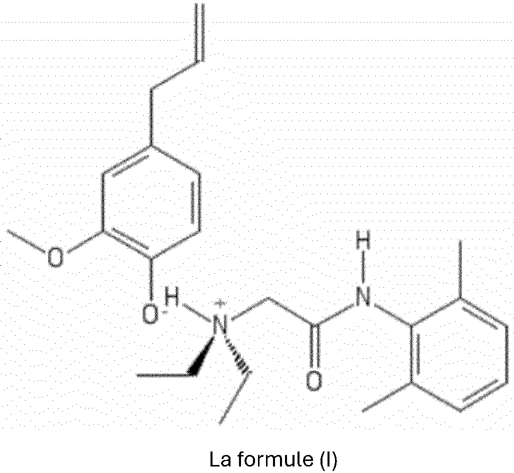

2. La combinaison selon la revendication 1 en tant que principe actif/fort agent antimicrobien pour le traitement des infections des voies respiratoires chez l'homme, causées par des agents pathogènes résistants aux antibiotiques tels que Pasteurella spp., Cryptococcus spp., Campylobacter spp., Microsporidia spp., Histoplasma capsulatum, Micrococcus spp., Candida spp. et d'autres bactéries, sous forme de liquide inhalable.
